# EUROPÄISCHE PATENTSCHRIFT

(11) **EP 1 008 578 B1**
(45) Veröffentlichungstag und Bekanntmachung des Hinweises auf die Patenterteilung: **04.09.2002**
(21) Anmeldenummer: 99123283.6
(22) Anmeldetag: 30.11.1999
(51) Int. Cl.: C07C 29/80, C07C 33/048

(54) **Verfahren zur destillativen Gewinnung von alpha-Ethinyl-Carbinolen**
Process for the recovery of alpha-ethynylcarbinols
Procédé pour la récupération d'alpha-éthynylcarbinols

(30) Priorität: 08.12.1998 DE 19856592
(43) Veröffentlichungstag der Anmeldung: 14.06.2000
(73) Patentinhaber: BASF AKTIENGESELLSCHAFT, 67056 Ludwigshafen (DE)
(72) Erfinder: Rahn, Ralf-Thomas, Dr., 68167 Mannheim (DE); Rust, Harald, 67435 Neustadt (DE); Rühl, Thomas, Dr., 67227 Frankenthal (DE); Henkelmann, Jochem, Dr., 68165 Mannheim (DE); Stutz, Susanne, Dr., 69469 Weinheim (DE)
(74) Vertreter: Isenbruck, Günter, Dr.

(56) Entgegenhaltungen:
- DE-A- 2 445 133
- US-A- 3 700 740
- CHEMICAL ABSTRACTS, vol. 093, no. 7, 18. August 1980 (1980-08-18) Columbus, Ohio, US; abstract no. 070912, ZARITSKII V V ET AL: "Study of the ethynylation of methyl vinyl ketone" XP002136090 & KHIM.-FARM. ZH. (KHFZAN,00231134);1980; VOL.14 (2); PP.84-7, Vses. Nauchno-Issled. Vitam. Inst.;Moscow; USSR

## Beschreibung

Die Erfindung betrifft ein Verfahren zur destillativen Gewinnung von α-Ethinyl-Carbinolen aus dem flüssigen Reaktionsgemisch der Addition von Acetylen an α,β-ungesättigte Carbonylverbindungen, sowie eine Verwendung des Verfahrens zur Gewinnung von Vinylbutinol.

α-Ethinyl-Carbinole sind Additionsprodukte von Acetylen an α,β-ungesättigte Carbonylverbindungen. Um während der Additionsreaktion die Polymerisationsneigung der Carbonylverbindung zu reduzieren, werden Alkali- bzw. Erdalkaliacetylide in der Regel bei tiefer Temperatur in einem dipolar-aprotischen Lösungsmittel als Reagenz eingesetzt. So hat es sich als günstig erwiesen, einen gemischten Natrium-Magnesium-Acetylid-Komplex oder Lithiumacetylid zu verwenden. Der Lithiumacetylidkomplex kann entweder direkt in einem Ether hergestellt werden, wobei dann zusätzlich die Verwendung eines Komplexbildners, beispielsweise Dimethylsulfoxid oder Dimethylacetamid, entsprechend der US 3,576,889, empfehlenswert ist oder das Lithiumacetylid wird in flüssigem Ammoniak hergestellt und nachfolgend Ammoniak gegen einen Ether ausgetauscht, entsprechend dem in CH-A-642 936 vorgeschlagenen Verfahren. Die Umsetzung mit der Carbonylverbindung erfolgt bei niedrigen Temperaturen; die CH-A-642 936 empfiehlt Temperaturen von -30 bis 20 °C, vorzugsweise von- 20 bis 10 °C. Nach erfolgter Addition wird das entstandene α-Ethinyl-Carbinolat hydrolisiert. Zur Aufarbeitung des Produktgemisches im Labormaßstab beschreibt die CH-A-642 936 die Trocknung der Etherphase mit Natriumsulfat und anschließende Destillation mit einer Vigreux-Kolonne.

Verfahren zur Aufarbeitung des Produktgemisches der Additionsreaktion von Acetylen an α,β-ungesättigte Carbonylverbindungen in großtechnischem Maßstab sind bislang nicht bekannt. Als problematisch erweisen sich hierbei zum einen das hohe Gefahrenpotential (die Zerfallsenergie von α-Ethinyl-Carbinolen liegt in Bereichen von oberhalb 1000 J/g) und zum anderen die Gewinnung von α-Ethinyl-Carbinolen aus wasserhaltigen Gemischen, da α-Ethinyl-Carbinole in der Regel mit Wasser als Heteroazeotrope abdestillieren, die sich zwar in zwei Phasen auftrennen, jedoch mit nur geringfügig abweichender Zusammensetzung.

Bei absatzweise durchgeführten Destillationen, wie sie beispielsweise in der CH-A-642 936 beschrieben sind, besteht zudem ein erhöhtes Gefahrenspotential durch die Anreicherung der leicht zersetzlichen α-Ethinyl-Carbinole im Kolonnensumpf.

Es ist Aufgabe der Erfindung, ein großtechnisches, kontinuierliches, destillatives Verfahren zur Gewinnung von α-Ethinyl-Carbinolen zur Verfügung zu stellen, daß sich durch eine hohe Wirtschaftlichkeit und durch eine hohe Sicherheit der Verfahrensführung auszeichnet.

Die Lösung geht aus von einem Verfahren zur destillativen Gewinnung von α-Ethinyl-Carbinolen aus dem flüssigen Reaktionsgemisch der Addition von Acetylen an α,β-ungesättigte Carbonylverbindungen in Gegenwart eines mit Wasser ein Azeotrop bildenden organischen Lösungsmittels, das einen um mindestens 5°C niedrigeren Siedepunkt gegenüber dem α-Ethinyl-Carbinol bzw. dem Azeotrop des α-Ethinyl-Carbinols mit Wasser, aufweist. Das erfindungsgemäße Verfahren ist dann dadurch gekennzeichnet, daß
a) die Destillation kontinuierlich erfolgt,
b) der Zulauf dem mittleren Teil einer Destillationskolonne zugeführt wird,
c) am Kolonnenkopf, der überwiegende Teil des Wassers azeotrop mit Lösungsmittel abdestilliert wird und
d) das Zielprodukt α-Ethinyl-Carbinol unterhalb des Kolonnenzulaufs gewonnen wird.

Es wurde gefunden, daß der überwiegende Teil des Wassers aus dem flüssigen Reaktionsgemisch der Addition von Acetylen an α,β-ungesättigte Carbonylverbindungen azeotrop mit dem Lösungsmittel über Kopf der Destillationskolonne ausgeschleust werden kann und somit das Problem der nicht unmittelbar durchzuführenden Auftrennung des Azeotrops Wasser/α-Ethinyl-Carbinol gelöst wird.

In der Additionsreaktion mit Acetylen kann grundsätzlich jede α,β-ungesättigte Carbonylverbindung der allgemeinen Formel 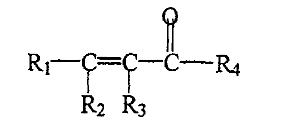 eingesetzt werden, worin R₁ und R₄ jeweils Wasserstoff oder ein unverzweigter oder ein- oder mehrfach verzweigter aliphatischer C₁-C₁₀-Rest oder ein unsubstituierter oder ein- oder mehrfach aliphatisch substituierter alicyclischer C₃-C₁₀-Rest oder R₁ und R₄ ein, beide Molekülteile verbindender gemeinsamer aliphatischer C₁-C₄-Rest und R₂ und R₃ jeweils Wasserstoff oder ein aliphatischer C₁-C₃-Rest sind. Bevorzugt werden als α,β-ungesättigte Carbonylverbindungen Acrolein, Methylvinylketon, Mesityloxid, β-Ionon, Pseudoionon, Citral oder Cyclopentenon eingesetzt.

Die Additionsreaktion wird in bekannter Weise durchgeführt, wobei ein Alkalioder Erdalkaliacetylid bei tiefer Temperatur in einem dipolar-aprotischen Lösungsmittel als Reagenz eingesetzt wird. Beispielsweise kann ein gemischter Natrium-Magnesium-Acetylidkomplex oder Lithiumacetylid verwendet werden. Der Lithium-Acetylidkomplex kann in bekannter Weise entweder unmittelbar in einem Ether hergestellt werden, wobei dann Zusätze eines Komplexbildners, wie Dimethylsulfoxid, Dimethylacetamid oder Dimethylformamid empfehlenswert sind oder das Lithiumacetylid wird in flüssigem Ammoniak hergestellt und nachfolgend Ammoniak gegen ein organisches Lösungsmittel, insbesondere gegen einen Ether, ausgetauscht. Für die Durchführung des vorliegenden Aufarbeitungsverfahrens sind dabei alle organischen Lösungsmittel geeignet, die mit Wasser ein Azeotrop bilden, das einen um mindestens 5°C, bevorzugt um mindestens 10°C, niedrigeren Siedepunkt gegenüber dem α-Ethinyl-Carbinol bzw. gegenüber dem Azeotrop des α-Ethinyl-Carbinols mit Wasser, aufweist. Dazu zählen insbesondere Ether, beispielsweise Diethylether, Tetrahydrofuran, Isopropylether, weiterhin aliphatische, cycloaliphatische oder aromatische Kohlenwasserstoffe, beispielsweise Pentan, Cyclohexan oder Toluol, sowie Alkohole, insbesondere Butanol.

Ein typisches Reaktionsgemisch der Addition von Acetylen an α,β-ungesättigte Carbonylverbindungen weist folgende Zusammensetzung auf:

| | |
|---|---|
| α-Ethinyl-Carbinol | 2 bis 40 Gew.-%, bevorzugt 5 bis 20 Gew.-%, |
| Wasser | 0,1 bis 10 Gew.-%, bevorzugt 1 bis 5 Gew.-%, |
| Hochsieder | 0 bis 5 Gew.-%, |
| Mittelsieder | 0 bis 2 Gew.-% |

Rest Lösungsmittel.

Als Hochsieder werden vorliegend Oligomere und Polymere der α,β-ungesättigten Carbonylverbindung bezeichnet; sie haben praktisch keinen Dampfdruck.

Als Mittelsieder werden vorliegend Substanzen mit einem Siedepunkt bezeichnet, der oberhalb des Siedepunkts des jeweiligen α-Ethinyl-Carbinols liegt. Mittelsieder können beispielsweise Nebenprodukte sein, die durch Reaktion der α,β-Carbonylverbindung mit dem α-Ethinyl-Carbinol gebildet werden.

Gemäß dem Verfahren nach der Erfindung wird das flüssige Reaktionsgemisch der Addition von Acetylen an α,β-ungesattigte Carbonylverbindungen kontinuierlich dem mittleren Teil einer Destillationskolonne zugeführt; d.h. daß die erfindungsgemäß eingesetzte Destillationskolonne einen Abtriebsteil aufweist.

Das Zielprodukt α-Ethinyl-Carbinol wird unterhalb des Kolonnenzulaufs gewonnen. Bei moderaten Reinheitsanforderungen oder bei niedrigen Gehalten an abzutrennenden Mittel- und Hochsiedern kann das Zielprodukt aus dem Sumpf abgezogen werden. In der Regel wird das Zielprodukt jedoch als Seitenabzug, bevorzugt auf der zweiten bis fünften Trennstufe, gerechnet von Sumpf, gewonnen.

Die Destillationskolonne unterliegt grundsätzlich keinen apparativen Einschränkungen. Prinzipiell kann jede Vakuumdestillationskolonne verwendet werden, wobei Kolonnen mit trennwirksamen Einbauten in Form von strukturierten Packungen bevorzugt sind. Besonders bevorzugt sind Kolonnen, die im mittleren und unteren Teil strukturierte Packungen aufweisen und in ihrem oberen Teil Böden, bevorzugt zwei bis drei Böden; am Kolonnenkopf kann ein Tropfenabscheider vorgesehen sein. Insbesondere für den Fall geringer Siedepunktsunterschiede zwischen dem Azetrop Lösungsmittel/Wasser und dem Azetrop α-Ethinyl-Carbinol/Wasser kann die Destillationskolonne als Trennwandkolonne ausgestaltet sein.

Die bevorzugten Betriebsbedingungen der Destillationskolonne sind:
Druck 10 mbar bis 1 bar absolut, bevorzugt 100 bis 700 mbar,
Trennstufenzahl 5 bis 50, bevorzugt 7 bis 20, besonders bevorzugt 8 bis 15.

Um noch mit Kühlwasser kondensieren zu können, wird zweckmäßigerweise der Kopfdruck der Destillationskolonne nur soweit abgesenkt, daß eine Kopfkondensationstemperatur von ca. 35°C nicht unterschritten wird. Wird beispielsweise Methyl-tert.-butylether als Lösungsmittel verwendet, so wird ein Kopfdruck von ca. 500 mbar eingestellt. Am Kopf der Destillationskolonne wird der überwiegende Teil des Wassers aus dem flüssigen Reaktionsgemisch azeotrop mit dem Lösungsmittel abdestilliert.

In vorteilhafter Weise werden Lösungsmittel eingesetzt, die mit Wasser ein Heteroazeotrop bilden, d.h. ein Azeotrop, das sich nach dem Kondensieren in zwei Phasen mit unterschiedlicher Zusammensetzung auftrennt. In diesem Fall wird das Kopfprodukt nach Kondensation einem Phasenscheider zugeführt und die organische Phase oder ein Teil derselben als Rücklauf auf die Destillationskolonne gegeben.

In bevorzugter Weise wird der Zulauf zum Phasenscheider unterkühlt, wodurch die Löslichkeit des Wassers in der organischen Phase reduziert und somit eine verstärkte Abtrennung von Wasser erreicht wird.

Erfindungsgemäß wird das Zielprodukt α-Ethinyl-Carbinol unterhalb des Kolonnenzulaufs gewonnen. In der Regel erfolgt der Abzug des Zielprodukts α-Ethinyl-Carbinol über einen Seitenabzug der Destillationskolonne. Im Kolonnensumpf fallen dann Hochsieder und Mittelsieder, neben geringen Mengen an α-Ethinyl-Carbinol an. Im Kolonnensumpf soll möglichst wenig Zielprodukt verloren gehen. Wegen der leichten Zersetzlichkeit der α-Ethinyl-Carbinole darf jedoch eine Maximaltemperatur nicht überschritten werden, die in der Regel bei etwa 50 Kelvin unterhalb der Onset-Zersetzungstemperatur liegt. So zersetzt sich beispielsweise reines Vinylbutinol ab ca. 170°C mit einer Energiefreisetzung von ca. 2000 J/g. Die Sumpftemperatur wird bei einer Verweilzeit im Stundenbereich auf maximal 100°C begrenzt, bei einer Verweilzeit im Minutenbereich auf maximal 120°C.

Gemäß einer bevorzugten Verfahrensvariante wird der Destillationskolonne ein Vorverdampfer vorgeschaltet, der bei reduziertem Druck, bevorzugt bei einem Druck von 10 mbar bis 1 bar absolut, besonders bevorzugt bei 100 bis 700 mbar, betrieben wird. Der Betriebsdruck des Vorverdampfers wird aus denselben Gründen, wie sie oben mit Bezug auf den Sumpf der Destillationskolonne ausgeführt wurden, unter Berücksichtigung der Zersetzlichkeit der α-Ethinyl-Carbinole, begrenzt. Zur Produktschonung werden Vorverdampfer nach dem Fallfilm-Prinzip oder besonders bevorzugt Dünnschichtverdampfer (d.h. Apparate mit niedrigen Verweilzeiten), eingesetzt. Der Dünnschichtverdampfer wird bevorzugt im Gleichstrom betrieben, d.h. der Brüden wird am unteren Ende des Apparats, in der Nähe des die Hochsieder enthaltenden Sumpfaustrags, abgezogen. Bei dieser Fahrweise ist der Restgehalt an α-Ethinyl-Carbinol im Hochsiederrückstand geringer als in der Gegenstromfahrweise.

Da im Sumpf der Destillationskolonne aufgrund der wegen der Zersetzlichkeit notwendigen Temperaturbegrenzung auch Zielprodukt, α-Ethinyl-Carbinol, abgezogen wird, ist es vorteilhaft, den Sumpfstrom zum Vorverdampfer zurückzuführen. Das erfindungsgemäße Verfahren wird vorteilhaft zur Gewinnung von Vinylbutinol eingesetzt. Bei der Gewinnung von Vinylbutinol wird beispielsweise bei einem Kolonnenvakuum von 500 mbar ein Sumpfprodukt von ca. 75 Gew.-% Vinylbutinol und ca. 25 Gew.-% Mittelsieder erhalten. Bei ca. 2 Gew.-% Mittelsiedern und ca. 18 Gew.-% Vinylbutinol im Zulauf zur Destillationskolonne gehen dadurch ca. 33 % des Vinylbutinols verloren. Der Verlust an Zielprodukt kann, wie angegeben, durch Rückführung des Sumpfstroms zum Vorverdampfer begrenzt werden.

Die Erfindung wird im folgenden anhand von Ausführungsbeispielen und einer Figur näher erläutert. Es zeigen
- Figur 1: die schematische Darstellung eines ersten Ausführungsbeispiels eines erfindungsgemäßen Verfahrens und
- Figur 2: die schematische Darstellung eines zweiten, bevorzugten Ausführungsbeispiels eines erfindungsgemäßen Verfahrens.

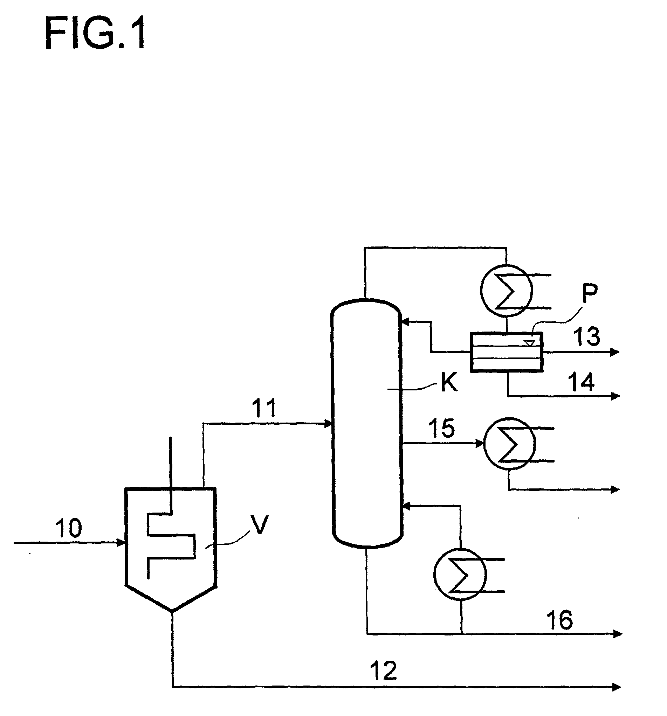

Im folgenden wird ein erstes Ausführungsbeispiel in Verbindung mit Figur 1 beschrieben:

400 g/h eines flüssigen Reaktionsgemisches der Addition von Acetylen an Methylvinylketon mit einer Zusammensetzung von 78 % Methyl-tert.-butylether als Lösungsmittel (im folgenden abgekürzt mit MTBE bezeichnet), 17 Gew.-% Zielprodukt Vinylbutinol (im folgenden abgekürzt mit VBI bezeichnet), 3 Gew.-% Wasser, 2 Gew.-% Mittel- und Hochsieder wurden einem Dünnschichtverdampfer V zugeführt, der im Gleichstrom mit einer Ölbadtemperatur von 130°C und einem Druck von 500 mbar absolut betrieben wurde. Über den Verdampfersumpf wurden 10 g/h Hochsieder ausgeschleust (Strom 12); der Brüden (Strom 11) wurde auf die fünfte theoretische Trennstufe einer Destillationskolonne K mit strukturierten Packungen aus Metallgewebe und 10 theoretischen Trennstufen mit einem Phasenscheider P am Kopf und einem dampfförmigen Seitenabzug auf der dritten theoretischen Trennstufe (Strom 15), dem ein Kondensator nachgeschaltet war, geführt. Die Destillationskolonne K wurde mit einem Kopfdruck von 500 mbar absolut und einer Kopftemperatur von 33°C betrieben. Der kondensierte Brüden wurde einem Phasenscheider (P) zugeführt. Die schwerere Wasserphase (Strom 14) wurde abgezogen, die organische Phase, die praktisch aus reinem MTBE mit ca. 1,5 Gew.-% gelöstem Wasser bestand, wurde im Verhältnis 5:1 wieder auf die Kolonne gegeben. Es wurde ein Destillatstrom von 322 g/h wassergesättigtem MTBE (Strom 13) und 6 g/h MTBE-gesättigtes Wasser (Strom 14) abgezogen. Bei einer Temperatur im Seitenabzug von 100°C wurden 62 g/h Vinylbutinol (Strom 15) mit einer Zusammensetzung von 99 Gew.-% Vinylbutinol, 1 Gew.-% MTBE und 150 ppm Mittelsieder dampfförmig entnommen und einem Kondensator zugeführt.

Im Kolonnensumpf wurden bei einer Temperatur von 107°C 5 g/h vinylbutinolhaltiges Hochsiedergemisch abgezogen und ausgeschleust (Strom 16), Die insgesamt ausgeschleuste Menge an Hochsiedern (Summe der Ströme 12 und 16) betrug ca. 15 g pro Stunde. Es wurden 62 g/h Vinylbutinol gewonnen, d.h. die Destillationsausbeute an Vinylbutinol betrug 91,2 %.

Die Zusammensetzung der Ströme 10 bis 16 ist in der nachfolgenden Tabelle 1 aufgeführt:

**Tabelle 1:**

| Strom Nr. | 10 | 11 | 12 | 13 | 14 | 15 | 16 |
|---|---|---|---|---|---|---|---|
| Massenstrom (g/h) | 400 | 390 | 10 | 322 | | 62 | 5 |
| Massenanteil | | | | | | | |
| MTBE | 0,78 | 0,80 | 0,09 | 0,985 | 0,02 | 0,01 | 26 ppm |
| H₂O | 0,03 | 0,03 | 0,01 | 0,015 | 0,98 | 18 ppm | < 1 ppm |
| VBI | 0,17 | 0,17 | 0,09 | < 1 ppm | < 1 ppm | 0,99 | 0,70 |
| HS | 0,02 | < 1 ppm | 0,77 | < 1 ppm | < 1 ppm | < 1 ppm | 18 ppm |
| MS | <0,01 | < 0,01 | 0,04 | < 1 ppm | < 1 ppm | 150 ppm | 0,30 |

**Tabelle 2:**

| Strom Nr. | 10 | 11 | 12 | 13 | 14 | 15 | 16 |
|---|---|---|---|---|---|---|---|
| Massenstrom (g/h) | 400 | 414 | 12 | 322 | | 66 | 26 |
| Massenanteil | | | | | | | |
| MTBE | 0,78 | 0,75 | 0,08 | 0,985 | 0,02 | 0,01 | 22 ppm |
| H₂O | 0,03 | 0,03 | 0,01 | 0,015 | 0,98 | 17 ppm | < 1 ppm |
| VBI | 0,17 | 0,20 | 0,10 | < 1 ppm | < 1 ppm | 0,99 | 0,70 |
| HS | 0,02 | < 1 ppm | 0,65 | < 1ppm | < 1 ppm | < 1ppm | 4 ppm |
| MS | < 0,01 | 0,02 | 0,16 | < 1ppm | < 1 ppm | 481 ppm | 0,30 |
| mit MTBE = Methyl-tert.-butylether VBI = Vinylbutinol HS = Hochsieder MS = Mittelsieder | | | | | | | |

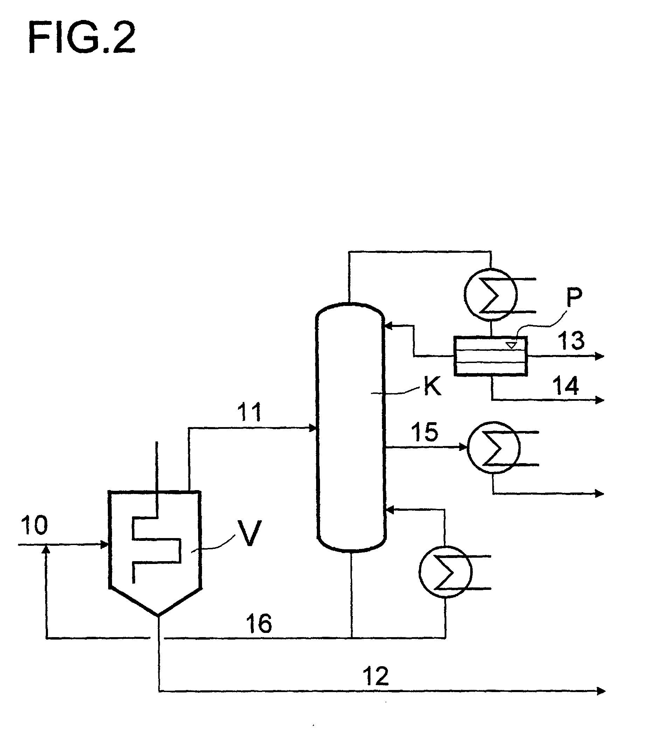

Ausführungsbeispiel 2 wird im Folgenden in Verbindung mit Figur 2 beschrieben:

400 g/h des Reaktionsgemisches von Acetylen mit Methylvinylketon mit einer Zusammensetzung von 78 % MTBE, 17 Gew.-% VBE, 3 Gew.-% Wasser sowie 2 Gew.-% Mittelsieder wurden einem Dünnschichtverdampfer (V) zugeführt (Strom 10). Im stationären Betrieb wurden zusätzlich zum genannten Zulauf noch 30 g/h zum Strom der Destillationskolonne K (Strom 16) in den Dünnschichtverdampfer V gefahren, der im Gleichstrom mit einer Ölbadtemperatur von 130°C und einem Druck von 500 mbar absolut betrieben wurde. Über den Verdampfersumpf wurden 12 g/h Hochsieder (Strom 12) ausgeschleust. Der Brüden wurde kondensiert und auf die fünfte theoretische Trennstufe einer Destillationskolonne K mit 10 theoretischen Trennstufen gepumpt, die am Kopf mit einem Phasenscheider P ausgestattet war und auf der dritten theoretischen Trennstufe einen dampfförmigen Seitenabzug aufwies, dem ein Kondensator nachgeschaltet war. Die Destillationskolonne K wurde mit einem Kopfdruck von 500 mbar absolut betrieben, die Kopftemperatur betrug 33°C. Der kondensierte Brüden wurde einem Phasenscheider P zugeführt. Die schwerere Wasserphase (Strom 14) wurde ausgeschleust. Die organische Phase, die aus praktisch reinem MTBE mit ca. 4 Gew.-% Wasser bestand, wurde im Verhältnis 5:1 wieder auf die Kolonne gegeben. Es wurde ein Destillatstrom von 322 g/h wassergesättigtem MTBE (Strom 13) und 6 g/h MTBE gesättigtes Wasser (Strom 14) abgezogen. Bei einer Temperatur im Seitenabzug von 100°C wurden 66 g/h VBI (Strom 15) mit einer Zusammensetzung von 99 Gew.-% VBI, 1 Gew.-% MTBE und 481 ppm Mittelsieder dampfförmig entnommen und einem Kondensator zugeführt. Im Kolonnensumpf wurde bei einer Temperatur von 107°C ein vinylbutinolhaltiges Hochsiedergemisch abgezogen (Strom 16) und in die Verdampfungsstufe zurückgeführt. Mit Rückführung des Hochsiederstroms der Destillation in den Vorverdampfer betrug die ausgeschleuste Hochsiedermenge nur 12 g/h. Es wurden 66 g/h Zielprodukt Vinylbutinol gewonnen. Die Destillationsausbeute an Vinylbutinol betrug 97,1 %.

## Patentansprüche

1. Verfahren zur destillativen Gewinnung von α-Ethinyl-Carbinolen aus dem flüssigen Reaktionsgemisch der Addition von Acetylen an α,β-ungesättigte Carbonylverbindungen in Gegenwart eines mit Wasser ein Azeotrop bildenden organischen Lösungsmittels, das einen um mindestens 5°C niedrigeren Siedepunkt gegenüber dem α-Ethinyl-Carbinol bzw. dem Azeotrop des α-Ethinyl-Carbinols mit Wasser, aufweist, **dadurch gekennzeichnet, daß**
a) die Destillation kontinuierlich erfolgt,
b) der Zulauf (11) dem mittleren Teil einer Destillationskolonne (K) zugeführt wird,
c) am Kolonnenkopf der überwiegende Teil des Wassers azeotrop mit dem Lösungsmittel abdestilliert wird und
d) das Zielprodukt α-Ethinyl-Carbinol unterhalb des Kolonnenzulaufs (11) gewonnen wird.

2. Verfahren nach Anspruch 1, **dadurch gekennzeichnet, daß** das Zielprodukt α-Ethinyl-Carbinol als Seitenabzug gewonnen wird.

3. Verfahren nach Anspruch 1 oder 2, **gekennzeichnet durch** folgende Betriebsparameter der Destillationskolonne (K):
Druck 10 mbar bis 1 bar absolut, bevorzugt 100 bis 700 mbar,
Trennstufenzahl 5 bis 50, bevorzugt 7 bis 20, besonders bevorzugt 8 bis 15.

4. Verfahren nach einem der Ansprüche 1 bis 3, **dadurch gekennzeichnet, daß** das Kopfprodukt der Destillationskolonne (K) einem Phasenscheider (P) zugeführt wird und daß die organische Phase oder ein Teil derselben als Rücklauf auf die Destillationskolonne (K) gegeben wird.

5. Verfahren nach Anspruch 4, **dadurch gekennzeichnet, daß** der Zulauf zum Phasenscheider (P) unterkühlt wird.

6. Verfahren nach einem der Ansprüche 1 bis 5, **dadurch gekennzeichnet, daß** der Destillationskolonne ein Vorverdampfer (V) vorgeschaltet ist, der bei reduziertem Druck, bevorzugt bei einem Druck von 10 mbar bis 1 bar absolut, besonders bevorzugt bei 100 bis 700 mbar absolut, betrieben wird.

7. Verfahren nach Anspruch 6, **dadurch gekennzeichnet, daß** der Vorverdampfer (V) ein Dünnschichtverdampfer ist.

8. Verfahren nach Anspruch 7, **dadurch gekennzeichnet, daß** der Dünnschichtverdampfer im Gleichstrom betrieben wird.

9. Verfahren nach einem der Ansprüche 6 bis 8, **dadurch gekennzeichnet, daß** das Sumpfprodukt (16) der Destillationskolonne (K) in den Vorverdampfer (V) zurückgeführt wird.

10. Verwendung des Verfahrens nach einem der Ansprüche 1 bis 9 zur destillativen Gewinnung von Vinylbutinol.

## Claims

1. A process for isolating α-ethynyl carbinols from the liquid reaction mixture from the addition of acetylene onto α,β-unsaturated carbonyl compounds by distillation in the presence of an organic solvent which forms as azeotrope with water and has a boiling point which is at least 5°C lower than that of the α-ethynyl carbinol or the azeotrope of the α-ethynyl carbinol with water, which comprises
a) carrying out the distillation continuously,
b) introducing the feed stream (11) in the middle section of a distillation column (K),
c) azeotropically distilling off the major part of the water with the solvent at the top of the column and
d) taking off the α-ethynyl carbinol target product below the feed point (11) to the column.

2. A process as claimed in claim 1, wherein the α-ethynyl carbinol target product is taken off at a side offtake.

3. A process as claimed in claim 1 or 2, wherein the operating parameters of the distillation column (K) are as follows:
pressure: from 10 mbar to 1 bar absolute, preferably from 100 to 700 mbar,
number of theoretical plates: from 5 to 50, preferably from 7 to 20, particularly preferably from 8 to 15.

4. A process as claimed in any of claims 1 to 3, wherein the top product from the distillation column (K) is fed to a phase separator (P) and the organic phase or part thereof is returned as runback to the distillation column (K).

5. A process as claimed in claim 4, wherein the feed to the phase separator (P) is undercooled.

6. A process as claimed in any of claims 1 to 5, wherein a prevaporizer (V) which is operated under reduced pressure, preferably at a pressure of from 10 mbar to 1 bar absolute, particularly preferably from 100 to 700 mbar absolute, is installed upstream of the distillation column.

7. A process as claimed in claim 6, wherein the prevaporizer (V) is a thin film evaporator.

8. A process as claimed in claim 7, wherein the thin film evaporator is operated in cocurrent.

9. A process as claimed in any of claims 6 to 8, wherein the bottoms (16) from the distillation column (K) are recirculated to the prevaporizer (V).

10. The use of a process as claimed in any of claims 1 to 9 for isolating vinylbutynol by distillation.

## Revendications

1. Procédé pour l'obtention par distillation d'α-éthynyl-carbinols à partir d'un mélange réactionnel liquide résultant de l'addition d'acétylène à des composés carbonylés α,β-insaturés en présence d'un solvant organique formant un azéotrope avec l'eau, ledit solvant présentant un point d'ébullition inférieur d'au moins 5°C à celui de l'α-éthynyl-carbinol ou de l'azéotrope de l'α-éthynyl-carbinol avec l'eau, **caractérisé par le fait que**
a) la distillation est conduite en continu,
b) l'introduction (11) est effectuée dans la partie médiane d'une colonne de distillation (K),
c) en tête de colonne la majeure partie de l'eau est extraite par distillation sous forme d'azéotrope avec le solvant, et
d) le produit visé α-éthynyl-carbinol est obtenu en dessous de l'introduction dans la colonne (11).

2. Procédé selon la revendication 1, **caractérisé par le fait que** le produit visé α-éthynyl-carbinol est obtenu sous forme d'un soutirage latéral.

3. Procédé selon la revendication 1 ou 2, **caractérisé par** les paramètres de fonctionnement de la colonne de distillation (K) suivants:
pression 10 mbar à 1 bar absolus, de préférence 100 à 700 mbar,
nombre de niveaux de séparation 5 à 50, de préférence 7 à 20, tout particulièrement 8 à 15.

4. Procédé selon l'une des revendications 1 à 3, **caractérisé par le fait que** le produit de tête de la colonne de distillation (K) est envoyé dans un séparateur de phases (P) et que la phase organique ou une partie de celle-ci est introduite sous forme de reflux dans la colonne de distillation (K).

5. Procédé selon la revendication 4, **caractérisé par le fait que** l'addition est surfondue dans le séparateur de phases (P).

6. Procédé selon l'une des revendications 1 à 5, **caractérisé par le fait qu'**avant la colonne de distillation est intercalé un pré-évaporateur (V), qui est mis en fonctionnement sous pression réduite, de préférence sous une pression de 10 mbar à 1 bar absolus, tout particulièrement avantageusement de 100 à 700 mbar absolus.

7. Procédé selon la revendication 6, **caractérisé par le fait que** le pré-évaporateur (V) est un évaporateur en couche mince.

8. Procédé selon la revendication 7, **caractérisé par le fait que** l'évaporateur en couche mince est mis en fonctionnement en courant parallèle.

9. Procédé selon l'une des revendications 6 à 8, **caractérisé par le fait que** le produit pâteux (16) de la colonne de distillation (K) est renvoyé dans le pré-évaporateur (V).

10. Utilisation du procédé selon l'une des revendications 1 à 9 pour l'obtention de vinylbutynol par distillation.
